(19) Europäisches Patentamt
European Patent Office
Office européen des brevets

(11) Publication number: **0 307 519 B1**

(12) **EUROPEAN PATENT SPECIFICATION**

(45) Date of publication of patent specification: **17.04.91**

(51) Int. Cl.⁵: **C07B 37/04**, C07C 69/94, C07C 43/29, C07C 253/00, C07C 67/343, C07C 41/30, C07C 255/00

(21) Application number: **87307569.1**

(22) Date of filing: **26.08.87**

(54) **Perfluoroalkylation process.**

(43) Date of publication of application:
**22.03.89 Bulletin 89/12**

(45) Publication of the grant of the patent:
**17.04.91 Bulletin 91/16**

(84) Designated Contracting States:
**AT BE CH DE FR GB IT LI LU NL SE**

(56) References cited:
**US-A- 4 590 010**

**CHEMISTRY LETTERS, no. 12, December 1981, pages 1719-1720, The Chemical Society of Japan; K. MATSUI et al.: "A convenient trifluoromethylation of aromatic halides with sodium trifluoroacetate"**

(73) Proprietor: **ETHYL CORPORATION**
**Ethyl Tower 451 Florida Boulevard**
**Baton Rouge Louisiana 70801(US)**

(72) Inventor: **Lin, Ronny Wen-Long**
**7335 President Drive**
**Baton Route, LA 70817(US)**
Inventor: **Davidson, Robert Ira**
**13156 Briargrove Avenue**
**Baton Route, LA 70810(US)**

(74) Representative: **Collier, Jeremy Austin Grey et al**
**J.A.Kemp & Co., 14 South Square, Gray's Inn**
**London WC1R 5LX(GB)**

**EP 0 307 519 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to perfluoroalkylaromatic compounds and more particularly to processes for preparing them.

As disclosed in McLoughlin et al., Tetrahedron , Vol. 25, pp. 5921-5940, 1969, Kobayashi et al., Tetrahedron Letters , No. 42, pp. 4071-4072, 1979, Gassman et al., Tetrahedron Letters , Vol. 26, No. 43, pp. 5243-5246, 1985, and U. S. Patents 3,408,411 (McLoughlin et al.) and 4,439,617 (Sestanj et al.), it is known that perfluoroalkylaromatic compounds are apt to be useful as biologically-active compounds, surfactants, coatings, sealants, dyestuffs, and alkyd-type resins, and they can be prepared in various ways. Matsui et al., Chemistry Letters , 1981, pp. 1719-1720, teach that aromatic halides may be trifluoromethylated with sodium trifluoroacetate in the presence of cuprous iodide and a dipolar aprotic solvent. U. S. patent 4,590,010 (Ramachandran et al.), filed April 18, 1985, discloses the use of the technique of Matsui et al. in trifluoromethylating 6-alkoxy-5-halo-1-cyanonaphthalenes and hydrocarbyl 6-alkoxy-5-halo-1-naphthoates.

An object of this invention is to provide a novel process for preparing perfluoroalkylaromatic compounds, particularly trifluoromethylaromatic compounds.

Another object is to provide a process for preparing the trifluoromethyl aromatic compounds utilizing a trifluoromethylating agent that is more selective than the prior art sodium trifluoroacetate and can be used in smaller amounts and require less reaction time.

These and other objects are obtained by reacting an aromatic bromide or iodide with at least about one equivalent of an organic acid salt corresponding to the formula set forth below in the presence of cuprous iodide and a dipolar aprotic solvent:

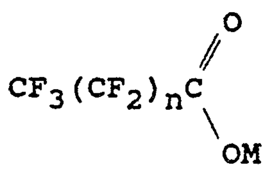

[I]

in which M is potassium or $-NR^1R^2R^3R^4$; $R^1$, $R^2$, $R^3$ and $R^4$ are the same or different alkyl groups containing from 1 to 20 carbon atoms, and n is 0 when M is $-NR^1R^2R^3R^4$ and 0 or an interger of at least 1 when M is potassium.

In the case of preparing the trifluoromethyl compounds it is sometimes useful to employ in the reaction a phase transfer catalyst so as to increase the conversion to the desired compound.

Aromatic halides utilizable in the practice of the invention are substituted and unsubstituted aromatic iodides and bromides wherein any substituents are inert substituents (i.e., substituents that do not prevent the reaction from occurring) such as alkyl, alkoxy, alkylthio, aryl, aryloxy, arylthio, cyano, nitro, acylamino, alkylamino, tertiary amino, sulfonamido, sulfone, sulfonyl, phosphino, perfluoroalkyl, chloro, fluoro, ester, aldehyde, ketone, acetal, and sulfono groups, and the aromatic ring may be a carbocyclic ring such as a benzene, naphthalene, and anthracene, ring or a five- or six-membered heterocyclic ring having aromatic character, e.g., a pyridine, quinoline, isoquinoline, thiophene, pyrrole, or furan ring. Exemplary of such compounds are iodobenzene, 3-iodotoluene, 4-chloroiodobenzene, 4-iodomethoxybenzene, 1-iodonaphthalene, 3-iodoaniline, 1-iodo-3-nitrobenzene, 2-iodothiophene, 4-iodoisoquinoline, 2-iodopyridine, 3-iodoquinoline, and the corresponding bromides.

In a preferred embodiment of the invention, the aromatic halide is a halonaphthalene corresponding to the formula:

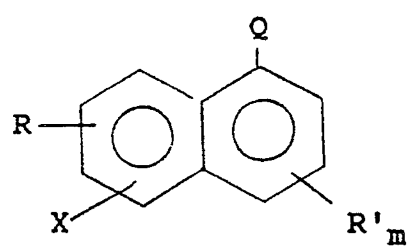

wherein R and R′ are independently selected from chloro, fluoro, nitro, hydroxy, and alkyl and alkoxy substituents containing 1-6 carbons; Q is -CN or -COOR″; R″ is saturated hydrocarbyl; X is bromo or iodo; and m is 0 or 1.

The halocyanonaphthalenes and halonaphthoates utilizable in the practice of the invention may be any compounds corresponding to the above halonaphthalene formula, but they are preferably compounds wherein m is 0, X is in the 5-position, and R is an alkyl or alkoxy substituent in the 6-position. When the R and R′ substituents are alkyl or alkoxy, they are generally straight-chain groups of 1-3 carbons or branched-chain groups of three or four carbons, such as methyl, ethyl, propyl, 1-methylethyl, butyl, 2-methylpropyl, 1,1-dimethylethyl, and the corresponding alkoxy groups, although, as indicated above, larger groups such as hexyl and hexanoxy are also utilizable. When the halonaphthalene is an ester, R″ may be any saturated hydrocarbyl group (i.e., a hydrocarbyl group that is free of aliphatic unsaturation) but is preferably an alkyl, cycloalkyl, aryl, alkaryl, or aralkyl group containing 1-10 carbons, e.g., methyl, ethyl, propyl, cyclohexyl, phenyl, tolyl, and benzyl. Particularly preferred halonaphthalenes are 6-alkoxy-5-bromo-1-cyanonaphthalenes, 6-alkoxy-5-iodo-1-cyanonaphthalenes, 6-alkoxy-5-bromo-1-naphthoates, and 6-alkoxy-5-iodo-1-naphthoates, especially those compounds wherein the alkoxy groups are methoxy.

The halonaphthoates are known compounds. The halocyanonaphthalenes are compounds that can be prepared by cyanating the appropriately substituted tetralone, e.g., 6-methoxytetralone, to form the appropriately substituted 1-cyano-3,4-dihydronaphthalene, e.g., 6-methoxy-1-cyano-3,4-dihydronaphthalene, aromatizing the product in any suitable manner, and brominating or iodinating the resultant substituted 1-cyanonaphthalene by known techniques.

In accordance with this invention, the halonaphthalene or other aromatic halide is reacted with at least one equivalent of an aromatic acid salt corresponding to Formula I as set forth below. When it is desired that the aromatic compound be trifluoromethylated, potassium trifluoroacetate or tetraalkyl ammonium trifluoroacetate is used.

Since there does not appear to be any maximum to the number of $CF_2$ groups that can desirably be incorporated into the aromatic molecule, the potassium salts employed in the reaction may be any compound corresponding to the formula $KOOC(CF_2)_nCF_3$ wherein n is 0 or an integer of at least 1, and it is generally the salt which contains the said number of $CF_2$ groups as is desired in the product. However, because of cost and availability factors, as well as the fact that the reaction typically permits the formation of at least some perfluoroalkylaromatic compound containing more $CF_2$ groups in the substituent than are present in the perfluoroalkanoate, the preferred reactants are those containing 1-16 $CF_2$ groups, such as potassium pentafluoropropionate, heptafluorobutyrate, nonafluorovalerate, tridecafluoroheptanoate, pentadecafluorooctanoate, heptadecafluorononanoate, and nondecafluorodecanoate. There does not appear to be any maximum to the amount of salt that may be employed. However, as a practical matter, the amount used is generally in the range of 1-20 equivalents, preferably at least 1.5 equivalents.

When tetralkylammonium trifluoroacetate is used, it may be any such compound wherein the alkyl groups, which may be the same or different and may be straight-chain or branched, contain 1-20 carbons, generally 1-12 carbons. Exemplary of such compounds are the tetramethyl, tetraethyl, tetrapropyl, tetrabutyl, tetrapentyl, tetrahexyl, tetraheptyl, methyltributyl, methyltrioctyl, methyltrialkyl($C_8$-$C_{10}$), butyltripropyl, heptyltriethyl, octyltriethyl, dodecyltrimethyl, dodecyltriethyl, tetradecyltrimethyl, and hexadecyltrimethylammonium trifluoroacetates, etc. The preferred trifluoroacetates are those wherein the alkyl groups contain 1-6 carbons, especially tetramethylammonium trifluoroacetate. When not available, the tetralkylammonium trifluoroacetates may be prepared by reacting trifluoroacetic acid with a carnonate, hydroxide, or other salt of the appropriate amine or amines.

When preparing the trifluoromethyl compounds, the amount of tetraalkylammonium trifluoroacetate or potassium trifluoroacetate reacted with the aromatic halide is not critical and may be a considerable excess, such as the amounts of sodium trifluoroacetate that have been employed in the past. However, since such large amounts of tetraalkylammonium and potassium salts are not required, the amount used is generally in the range of 1-3 equivalents, most commonly 1.5-2 equivalents.

Dipolar aprotic solvents that may be utilized include, e.g., N-methylpyrrolidone, N,N-dimethylformamide, N,N-dimethylacetamide, hexamethylphosphoric triamide, and dimethylsulfoxide, but the particular solvent employed does not appear to be critical except in the sense that it should have an appropriate boiling point for use at the reaction temperatures to be utilized. The solvent is used in solvent amounts, e.g., an amount such as to provide an organic solids concentration of up to about 15%.

The cuprous iodide may be employed in any suitable amount, generally an amount in the range of 0.5-5 equivalents.

The reaction is conducted by combining the ingredients in any convenient order and heating them at a suitable temperature, conveniently reflux temperature, to accomplish the desired perfluoroalkylation. Anhydrous conditions are preferably employed, and the temperature is generally in the range of 130-160° C., preferably 140-155° C.

The perfluoroalkylnaphthalene products of the reaction can be subjected to reactions such as those taught by Sestanj et al. Thus, e.g., (1) a (perfluoroalkyl)cyanonaphthalene or perfluoroalkylnaphthoate prepared by the perfluoroalkylation reaction may be hydrolyzed to the corresponding acid in the presence of a base such as sodium or potassium hydroxide, (2) the acid can be halogenated, e.g., by reaction with thionyl chloride, to form the corresponding acid halide, (2) the acid halide may be reacted with a saturated hydrocarbyl ester of an acid corresponding to the formula $ZNHCH_2COOH$ (e.g., methyl, ethyl, propyl, cyclohexyl, phenyl, tolyl, or benzyl sarcosinate, the corresponding esters of aminoacetic acids having other N-substituents containing 1-6 carbons, such as N-ethyl and N-propyl) to form an amide corresponding to the formula:

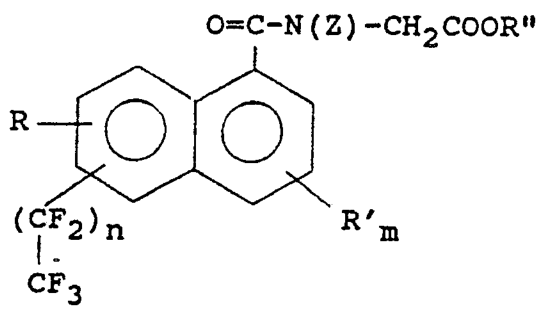

(3) the amide may be saponified to form the corresponding salt, then hydrolyzed to the corresponding acid, and then thiated, e.g., with phosphorus pentasulfide or the like, to form a thioamide corresponding to the formula:

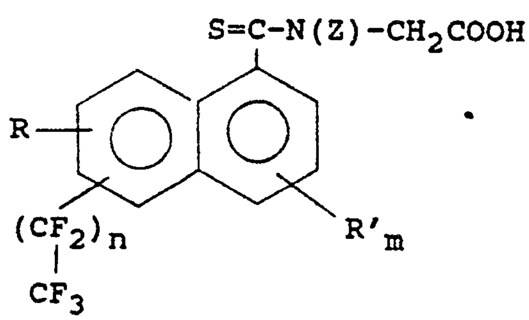

or (4) the thioamide may be prepared by thiating the amide and then subjecting the product to the saponification and hydrolysis steps.

In like manner, products obtained by trifluoromethylating the preferred halocyanonaphthalenes and halonaphthoates can be subjected to reactions such as those taught by Sestanj et al. in U.S. Patent 4,439,617. Thus, e.g., (1) a (trifluoromethyl)cyanonaphthalene or trifluoromethylnaphthoate prepared by the trifluoromethylation reaction may be hydrolyzed to the corresponding acid in the presence of a base such as sodium or potassium hydroxide, (2) the acid can be halogenated, e.g., by reaction with thionyl chloride, to form the corresponding acid halide, (2) the acid halide may be reacted with a saturated hydrocarbyl ester of an acid corresponding to the formula $ZNHCH_2COOH$ (e.g., methyl, ethyl, propyl, cyclohexyl, phenyl, tolyl, or benzyl sarcosinate, the corresponding esters of aminoacetic acids having other N-substituents containing 1-6 carbons, such as N-ethyl and N-propyl) to form an amide corresponding to the formula:

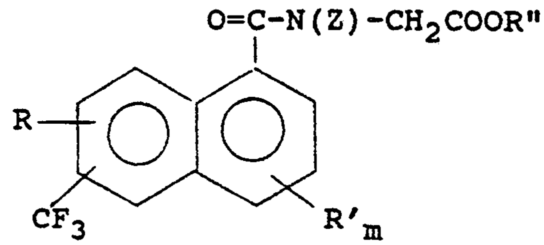

and (3) the amide may be thiated, e.g., with phosphorus pentasulfide or the like, and the product saponified and hydrolyzed to form a thioamide corresponding to the formula:

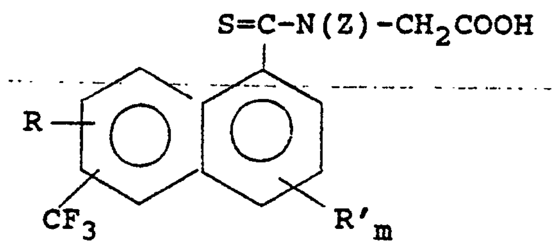

The invention is advantageous in that it provides a means of preparing perfluoroalkyl compounds useful in various applications, such as surfactants, coatings, sealants, resins, and dyestuffs, as well as biologically-active materials or precursors therefor. It also permits trifluoromethylaromatic compounds to be prepared in high yields from the corresponding aromatic bromides or iodides at a faster rate and with the use of less reagent than is required when sodium trifluoroacetate is employed. Also, the reaction is more selective than the sodium trifluoroacetate reaction, so the product is less contaminated with by-product. Additionally, the potassium trifluoroacetate and tetraalkylammonium trifluoroacetate reactions can be accomplished with higher concentrations of solids than are operable when sodium trifluoroacetate is used, and the reactor productivity can thus be increased considerably.

When using potassium trifluoroacetate, it is often desirable to use a phase transfer agent in the reaction. The phase transfer agent may be any such agent but is generally a tetraalkylammonium or tetralkylphosphonium halide, preferably a bromide or iodide. In these compounds the alkyl groups may be the same or different, straight-chain or branched, and contain 1-20 carbons, usually 1-12 carbons. Exemplary of such compounds are the tetramethyl, tetraethyl, tetrapropyl, tetrabutyl, tetrapentyl, tetrahexyl, tetraheptyl, methyltributyl, methyltrioctyl, methyltrialkyl($C_8$-$C_{10}$), butyltripropyl, heptyltriethyl, octyltriethyl, dodecyltrimethyl, dodecyltriethyl, tetradecyltrimethyl, and hexadecyltrimethylammonium bromides, the corresponding iodides, and the corresponding phosphonium halides. The preferred phase transfer agents are the halides in which the alkyl groups contain 1-6 carbon atoms, especially tetramethylammonium bromide. The amount of phase transfer agent employed does not appear to be critical but is conveniently in the range of 0.01-1, generally 0.1-0.3, equivalents. The use of a phase transfer agent will usually result in an increased conversion to trifluoromethyl aromatic compound.

The following examples are given to illustrate the invention and are not intended as a limitation thereof.

EXAMPLE I

A suitable reaction vessel was charged with 8.1 g of 6-methoxy-5-bromo-1-cyanonaphthalene, 11.8 g of CuI, 35 ml of toluene, and 55 ml of N,N-dimethylformamide. The reaction mixture was heated to 165° C. with concurrent azeotropic removal of toluene/ water (25 ml) and then maintained at 155° C. when 11.8 g of potassium pentafluoropropionate was added. The reaction was monitored by VPC. After five hours no starting material was detected and the reaction mixture was poured into 150 ml of water and 125 ml of methylene chloride. The two phases were filtered, after which the organic layer was separated, washed with brine, and concentrated in vacuo to provide a crude 6-methoxy-5-pentafluoroethyl-1-cyano-naphthalene (6-MPCN) having a purity of greater than 95%.

EXAMPLE II

The crude 6-MPCN product of Example I was dissolved in 135 ml of methanol and 40 ml of a potassium hydroxide solution (4.5 g of KOH in 40 ml of water) and heated to 125° C./70 psi for seven hours. The reaction mixture was then worked up and acidified to yield 6.6 g of 6-methoxy-5-pentafluoroethyl-1-naphthoic acid.

EXAMPLE III

A mixture of one molar proportion of 6-methoxy-5-iodo-1-cyanonaphthalene (6-MICN), 1.9 molar proportions of CuI, and 1.7 molar proportions of potassium trifluoroacetate was stirred into 10 molar proportions of toluene after which part of the toluene was stripped, 40 molar proportions of N,N-dimethylformamide (DMF) were added, and toluene and DMF were stripped until the temperature reached 149° C. The temperature was maintained at 149-150° C. for two hours, after which GC analysis showed that all of the 6-MICN had been converted and more than 98% had been converted to 6-methoxy-5-trifluoromethyl-1-cyanophthalene (6-MTCN).

EXAMPLE IV

A mixture of one molar proportion of 6-methoxy-5-bromo-1-cyanonaphthalene (6-MBCN), 2 molar proportions of CuI, and 1.7 molar proportions of potassium trifluoroacetate was stirred into 15 molar proportions of toluene, after which part of the toluene was stripped and 66 molar proportions of DMF were added. The reaction mixture was heated at 140-150° C. for about 3.5 hours, cooled, worked up, and subjected to GC analysis. the analysis showed substantially 100% conversion to 6-MTCN and a trace formation of by-product.

EXAMPLE V

Example IV was essentially repeated except that the amount of potassium trifluoroacetate used was 1.5 molar proportions, and 53 molar proportions of N,N-dimethylacetamide (DMAC) were substituted for the DMF. The GC analysis showed 99.77 area % of 6-MTCN.

COMPARATIVE EXAMPLE A

A mixture of one molar proportion of 6-MICN, 2.3 molar proportions of sodium trifluoroacetate, and 1.9 molar proportions of CuI was stirred into 146 molar proportions of toluene, after which about 82 molar proportions of the toluene were stripped at 111° C. Then 83 molar proportions of dry N,N-dimethylacetamide (DMAC) were added, and the mixture was heated while distilling over the remainder of the toluene until the temperature reached 152° C. Heating was stopped, another 0.7 molar proportion of sodium trifluoroacetate was added, and the reaction mixture was heated back up to 152° C. and stirred for 80 minutes at 152-155° C. to give a total reaction time of four hours. After cooling and workup, GC analysis showed the reaction mixture to contain 97.25 area % of the desired 6-MTCN.

COMPARATIVE EXAMPLE B

Comparative Example A was essentially repeated except that the 6-MICN was replaced with 6-MBCN and the total reaction time was 6 hours. GC analysis of the final reaction mixture showed 93.45 area % of 6-MTCN.

COMPARATIVE EXAMPLE C

Using the same general procedure as in Comparative Example A, one molar proportion of methyl 6-methoxy-5-bromo-1-naphthoate (MMBN) was reacted with 1.7 molar proportions of sodium trifluoroacetate in the presence of 2 molar proportions of CuI and 46 molar proportions of DMF, with an additional 0.95 molar proportion of sodium trifluoroacetate being added during the course of the reaction. The total reaction time was 5 hours. After workup, the desired methyl 6-methoxy-5-trifluoromethyl-1-naphthoate (MMTN) was isolated in a 76% yield.

EXAMPLE VI

Comparative Example C was essentially repeated except that the initial sodium trifluoroacetate was replaced with 1.75 molar proportions of potassium trifluoroacetate, no additional trifluoroacetate was added during the course of the reaction, and the reaction time was only 3.5 hours. GC analysis showed a conversion of 99.8%, and the isolated yield of product was 86%.

COMPARATIVE EXAMPLE D

A mixture of one molar proportion of 4-bromodiphenyl ether, 1.99 molar proportions of CuI, and 1.7 molar proportions of sodium trifluoroacetate was stirred into 14 molar proportions of toluene, after which part of the toluene was stripped, 46 molar proportions of DMF were added, and the reaction mixture was heated up to 149 °C. for three hours. Heating was stopped, another molar proportion of sodium trifluoroacetate was added, and the mixture was heated up to 149 °C. for two hours. GC analysis of the product showed 47.5 area % of the desired 4-trifluoromethyldiphenyl ether, 26.5 area % of perfluoroalkyl homologs, and 22.8 area % of unreacted 4-bromodiphenyl ether.

EXAMPLE VII

Comparative Example D was essentially repeated except that the initial sodium trifluoroacetate was replaced with 2.69 proportions of potassium trifluoroacetate, no additional trifluoroacetate was added during the course of the reaction, and the reaction time was 3.5 hours. GC analysis of the product showed 67 area % of the desired 4-trifluoromethyldiphenyl ether, 16.6 area % of perfluoroalkyl homologs, and 16.2 area % of unreacted 4-bromodiphenyl ether.

EXAMPLE VIII

A mixture of one molar proportion of 6-MBCN, 1.5 molar proportions of potassium trifluoroacetate, 2.1 molar proportions of CuI, and 0.1 molar proportion of tetramethylammonium bromide was stirred into 18 molar proportions of toluene, after which part of the toluene was stripped, 80 molar proportions of DMF were added, and toluene and DMF were stripped until the temperature reached about 155 °C. The reaction mixture was maintained at about 155 °C. for four hours, cooled, worked up, and subjected to HPLC analysis. The analysis showed 88.1% by weight of 6-MTCN and only 1.2% by weight of unreacted 6-MBCN.

COMPARATIVE EXAMPLE E

The above example was essentially repeated except that the tetramethylammonium bromide was not employed. The analysis of the product showed 88.0% by weight of 6-MTCN and 4.7% by weight of unreacted 6-MBCN.

EXAMPLE IX

A mixture of one molar proportion of 6-MBCN, 1.5 molar proportions of tetramethylammonium trifluoroacetate (TMATA), and 2.1 molar proportions of CuI was stirred into 18 molar proportions of toluene, after which part of the toluene was stripped, 80 molar proportions of DMF were added, and toluene and DMF were stripped until the temperature reached about 156 °C. The reaction mixture was maintained at

about 156° C. for two hours, cooled, worked up, and subjected to HPLC analysis. The analysis showed 87.1% by weight of 6-MTCN, 4.5% by weight of 6-MBCN, and no 6-methoxy-5-pentafluoroethyl-1-cyanonaphthalene (MPCN).

EXAMPLE X

Example IX was essentially repeated except that the TMATA was replaced with tetrabutylammonium trifluoroacetate. Due to the formation of some tetrabutylammonium bromide by-product, the HPLC analysis was normalized to show 82.3% by weight of 6-MTCN, 8.5% by weight of 6-MBCN, and no MPCN.

COMPARATIVE EXAMPLE F

Example IX was essentially repeated except that the TMATA was replaced with four molar proportions of sodium trifluoroacetate. The HPLC analysis showed 80.9% by weight of 6-MTCN, only a trace of 6-MBCN, and 4.9% by weight of MPCN.

Reference is made to our co-pending European application filed 26 August, 1987, Representative's reference number REB/31047/000, our reference wumber Case 5540, the contents of which are incorporated herein by reference and a copy of the specification for which is filed herewith.

## Claims

1. A process for preparing a perfluoroalkyl aromatic compound which comprises reacting an aromatic bromide or iodide in the presence of cuprous iodide and a dipolar aprotic solvent with an organic acid salt corresponding to the following formula:

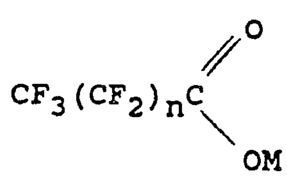

in which M is potassium or $-NR^1R^2R^3R^4$; $R^1$, $R^2$, $R^3$ and $R^4$ are the same or different alkyl groups containing from 1 to 20 carbon atoms, and n is 0 when M is $-NR^1R^2R^3R^4$ and 0 or an integer of at least 1 when M is potassium.

2. A process as claimed in Claim 1 in which the organic acid salt is a potassium perfluoroalkanate corresponding to the formula:

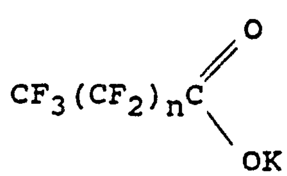

in which n is 0 or an integer of at least 1.

3. A process as claimed in Claim 2 in which the organic acid salt is potassium trifluoroacetate.

4. A process as claimed in Claim 3 in which the reaction is carried out in the presence of a phase transfer agent, e.g. a tetraalkylammonium or phosphonium halide.

5. A process as claimed in Claim 1 in which the organic acid salt is tetraalkylammonium trifluoroacetate.

6. A process as claimed in any one of Claims 2, 4 and 5 in which the aromatic bromide or iodide is a halonaphthalene corresponding to the formula:

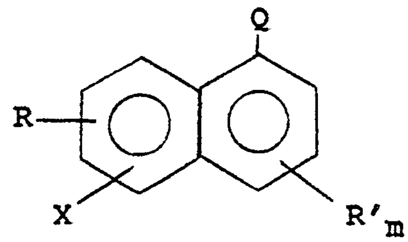

wherein R and R′, are independently selected from chloro, fluoro, nitro, hydroxy, and alkyl and alkoxy substituents containing 1-6 carbons; Q is -CN or -COOR″; R″ is saturated hydrocarbyl; X is bromo or iodo; and m is 0 or 1.

7. A process as claimed in Claim 6 in which the halonaphthalene is a 6-alkoxy-5-bromo-1-cyanonaphthalene or a 6-alkoxy-5-iodo-1-cyanonaphthalene.

8. A process for preparing perfluoroalkyl aromatic compounds which comprises reacting a halonaphthalene corresponding to the formula:

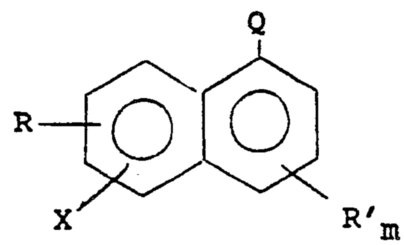

wherein R and R′ are independently selected from chloro, fluoro, nitro, hydroxy, and alkyl and alkoxy substituents containing 1-6 carbons; Q is -CN or -COOR″; R″ is saturated hydrocarbyl: X is bromo or iodo; and m is 0 or 1, with 1-3 equivalents or potassium trifluoroacetate in the presence of a dipolar aprotic solvent and 0.5-5 equivalents of cuprous iodide at 140-155°C. so as to form a trifluoromethylaromatic compound.

9. A process as claimed in Claim 8 in which the solvent is N,N-dimethylformamide or N,N-dimethylacetamide.

10. A process as claimed in Claim 8 or Claim 9 in which the halonaphthalene is a
6-alkoxy-5-bromo-1-cyanonaphthalene a
6-alkoxy-5-iodo-1-cyanonaphthalene, a
6-alkoxy-5-bromo-1-naphthoate, or a
6-alkoxy-5-iodo-1-naphthoate.

## Revendications

1. Procédé de préparation d'un composé perfluoralkyl-aromatique, qui consiste à faire réagir un bromure ou iodure aromatique en présence d'iodure cuivreux et d'un solvant aprotique dipolaire avec un sel d'acide organique correspondant à la formule suivante :

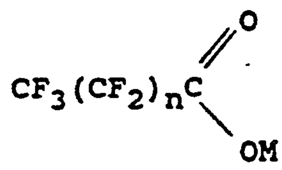

dans laquelle M est le potassium ou un groupe $-NR^1R^2R^3R^4$ ; $R^1$, $R^2$, $R^3$ et $R^4$ sont des groupes alkyle identiques ou différents contenant 1 à 20 atomes de carbone et n est égal à 0 lorsque M est un groupe $-NR^1R^2R^3R^4$ et à 0 ou à un nombre entier au moins égal à 1 lorsque M est le potassium.

2. Procédé suivant la revendication 1, dans lequel le sel d'acide organique est un perfluoralcanoate de potassium correspondant à la formule :

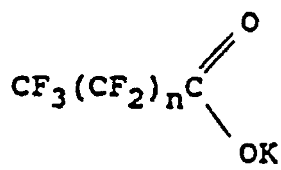

dans laquelle n est égal à 0 ou à un nombre entier au moins égal à 1.

3. Procédé suivant la revendication 2, dans lequel le sel d'acide organique est le trifluoracétate de potassium.

4. Procédé suivant la revendication 3, dans lequel la réaction est conduite en présence d'un agent de transfert de phase, par exemple un halogénure de tétraalkylammonium ou de phosphonium.

5. Procédé suivant la revendication 1, dans lequel le sel d'acide organique est un trifluoracétate de tétra-alkylammonium.

6. Procédé suivant l'une quelconque des revendications 2, 4 et 5, dans lequel le bromure ou iodure aromatique est un halogénonaphtalène répondant à la formule :

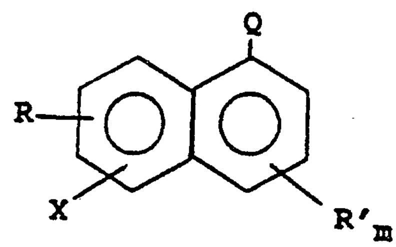

dans laquelle R et R' sont choisis, indépendamment, entre des substituants chloro, fluoro, nitro, hydroxy et des substituants alkyle et alkoxy contenant 1 à 6 atomes de carbone ; Q est un groupe -CN ou -COOR" ; R" est un groupe hydrocarbyle saturé ; X est un radical bromo ou iodo ; et m a la valeur 0 ou 1.

7. Procédé suivant la revendication 6, dans lequel l'halogénonaphtalène est un 6-alkoxy-5-bromo-1-cyanonaphtalène ou un 6-alkoxy-5-iodo-1-cyanonaphtalène.

8. Procédé de préparation de composés perfluoralkyl-aromatiques, qui consiste à faire réagir un halogénonaphtalène répondant à la formule :

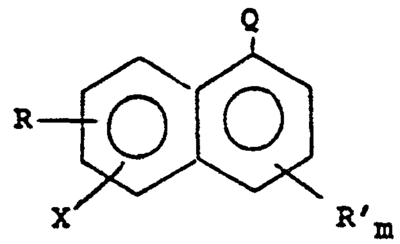

dans laquelle R et R' sont choisis, indépendamment, dans le groupe des substituants chloro, fluoro, nitro, hydroxy et des substituants alkyle et alkoxy contenant 1 à 6 atomes de carbone ; Q est un groupe -CN ou -COOR" ; R" est un groupe hydrocarbyle saturé ; X est un radical bromo ou iodo ; et m a la valeur 0 ou 1, avec 1 à 3 équivalents de triflouracétate de potassium en présence d'un solvant aprotique dipolaire et de 0,5 à 5 équivalents d'iodure cuivreux à 140-155° C de manière à former un composé trifluorométhyl-aromatique.

9. Procédé suivant la revendication 8, dans lequel le solvant est le N,N-diméthylformamide ou le N,N-diméthylacétamide.

10. Procédé suivant la revendication 8 ou la revendication 9, dans lequel l'halogénonaphtalène est un 6-alkoxy-5-bromo-1-cyanonaphtalène, un 6-alkoxy-5-iodo-1-cyanonaphtalène, un 6-alkoxy-5-bromo-1-naphtoate ou un 6-alkoxy-5-iodo-1-naphtoate.

## Ansprüche

1. Verfahren zur Herstellung einer Perfluoralkyl-aromatischen Verbindung, welches die Umsetzung eines aromatischen Bromids oder Jodids in Gegenwart von Kupfer(I)jodid und von einem dipolaren aprotischen Lösungsmittel mit dem Salz einer organischen Säure umfaßt, das der folgenden Formel entspricht:

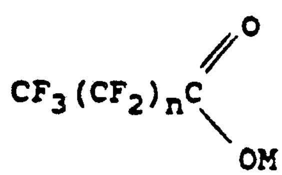

worin M Kalium oder $-NR^1R^2R^3R^4$ ist, $R^1$, $R^2$, $R^3$ und $R^4$ gleiche oder verschiedene Alkylgruppen sind, die 1 bis 20 Kohlenstoffatome enthalten, und n 0 ist, wenn M $-NR^1R^2R^3R^4$ ist, und 0 oder eine ganze Zahl von wenigstens 1 ist, wenn M Kalium ist.

2. Verfahren nach Anspruch 1, worin das Salz der organischen Säure ein Kaliumperfluoralkanoat entsprechend der folgenden Formel ist:

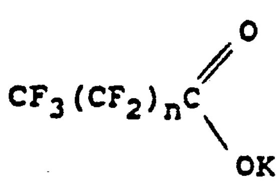

worin n 0 oder eine ganze Zahl von wenigstens 1 ist.

3. Verfahren nach Anspruch 2, worin das Salz der organischen Säure Kaliumtrifluoracetat ist.

4. Verfahren nach Anspruch 3, worin die Reaktion in Gegenwart eines Phasentransfermittels wie beispielsweise eines Tetraalkylammoniumhalogenids oder -phosphoniumhalogenids durchgeführt wird.

5. Verfahren nach Anspruch 1, worin das Salz der organischen Säure Tetraalkylammoniumtrifluoracetat ist.

6. Verfahren nach irgendeinem der Ansprüche 2, 4 und 5, worin das aromatische Bromid oder Jodid ein Halogennaphthalin entsprechend der folgenden Formel ist:

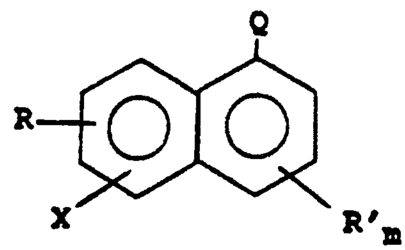

worin R und R' unabhängig voneinander gewählt sind unter Chlor-, Fluor-, Nitro-, Hydroxy- und Alkyl- sowie Alkoxy-Substituenten, die 1 bis 6 Kohlenstoffatome enthalten, Q -CN oder -COOR" ist, R" ein gesättigter Hydrocarbyl-Rest ist; X Brom oder Jod ist; und m 0 oder 1 ist.

7. Verfahren nach Anspruch 6, worin das Halogennaphthalin ein 6-Alkoxy-5-brom-1-cyanonaphthalin oder ein 6-Alkoxy-5-jod-1-cyanonaphthalin ist.

8. Verfahren zur Herstellung von Perfluoralkyl-aromatischen Verbindungen, welches die Umsetzung eines Halogennaphthalins, das der Formel

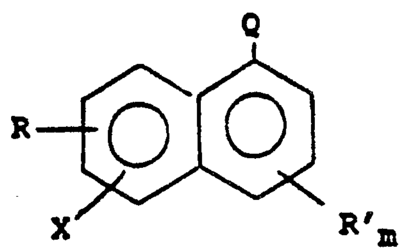

entspricht,
worin R und R' unabhängig voneinander gewählt sind unter Chlor-, Fluor-, Nitro-, Hydroxy- und Alkyl- sowie Alkoxy-Substituenten, die 1 bis 6 Kohlenstoffatome enthalten; Q -CN oder -COOR" ist; R" ein gesättigter Hydrocarbyl-Rest ist; X Brom oder Jod ist; und m 0 oder 1 ist, mit 1 bis 3 Equivalenten Kaliumtrifluoracetat in Gegenwart eines dipolaren aprotischen Lösungsmittels und 0,5 bis 5 Equivalenten Kupfer(I)jodid bei 140 bis 155 °C in der Weise umfaßt, daß eine Trifluormethyl-aromatische Verbindung gebildet wird.

9. Verfahren nach Anspruch 8, worin das Lösungsmittel N,N-Dimethylformamid oder N,N-Dimethylacetamid ist.

10. Verfahren nach Anspruch 8 oder Anspruch 9, worin das Halogennaphthalin ein 6-Alkoxy-5-brom-1-cyanonaphthalin, ein 6-Alkoxy-5-Jod-1-cyanonaphthalin, ein 6-Alkoxy-5-brom-1-naphthoat oder ein 6-Alkoxy-5-jod-1-naphthoat ist.